(19) Europäisches Patentamt / European Patent Office / Office européen des brevets

(11) **EP 3 002 585 A1**

(12) **EUROPEAN PATENT APPLICATION**

(43) Date of publication:
**06.04.2016 Bulletin 2016/14**

(51) Int Cl.:
**G01N 33/00** (2006.01)    **A61B 5/08** (2006.01)
**G01N 33/497** (2006.01)

(21) Application number: **15187012.8**

(22) Date of filing: **28.09.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **01.10.2014 JP 2014203138**

(71) Applicant: **Tanita Corporation
Tokyo (JP)**

(72) Inventors:
• **KASAHARA, Yasuhiro
  Itabashi-ku, Tokyo (JP)**
• **KOBAYASHI, Kotaku
  Itabashi-ku, Tokyo (JP)**
• **MINAGAWA, Naotaka
  Itabashi-ku, Tokyo (JP)**
• **KODAMA, Miyuki
  Itabashi-ku, Tokyo (JP)**
• **AZUMA, Shigeharu
  Itabashi-ku, Tokyo (JP)**
• **SANO, Ayumi
  Itabashi-ku, Tokyo (JP)**

(74) Representative: **Grünecker Patent- und
Rechtsanwälte
PartG mbB
Leopoldstraße 4
80802 München (DE)**

(54) **GAS MEASUREMENT APPARATUS**

(57)    A gas measurement apparatus includes: a body unit including a delivery port in which a user's breath is delivered, a gas sensor connected to the inside of the delivery port, and an absorbent housing unit that houses an absorbent; a cover unit capable of being operated to be slid in a predetermined direction with respect to the body unit and thereby being set to one of an open state in which the delivery port and the absorbent housing unit are exposed and a closed state in which the delivery port and the absorbent housing unit are cut off from an outside air, wherein at least in the closed state, a space in which the gas sensor is provided and the inside of the absorbent housing unit are connected to each other.

FIG. 1

EP 3 002 585 A1

**Description**

BACKGROUND

Field of the Invention

The present invention relates to a gas measurement apparatus.

Background

In the related art, gas measurement apparatuses that measure the concentration of a detection target gas or detect the presence or absence of a detection target gas are known (for example, refer to Japanese Patent Application, Publication No. JP2014-163671A). In such apparatuses, semiconductor sensors may be used that come into contact with various gases which can be included in the air to thereby be contaminated or that are deteriorated due to various causes.

SUMMARY

However, in gas measurement apparatuses of the related art, there are cases in which the operability for a user and the protection for a sensor are not sufficient.

An object of an aspect of the present invention is to provide a gas measurement apparatus capable of improving operability for a user and preventing contamination or degradation of a semiconductor sensor.

An aspect of the present invention is a gas measurement apparatus including: a body unit including a delivery port in which a user's breath is delivered, a gas sensor connected to the inside of the delivery port, and an absorbent housing unit that houses an absorbent; a cover unit capable of being operated to be slid in a predetermined direction with respect to the body unit and thereby being set to one of an open state in which the delivery port and the absorbent housing unit are exposed and a closed state in which the delivery port and the absorbent housing unit are cut off from an outside air, wherein at least in the closed state, a space in which the gas sensor is provided and the inside of the absorbent housing unit are connected to each other.

According to an aspect of the present invention, it is possible to improve operability for a user and to prevent contamination or degradation of a semiconductor sensor.

BRIEF DESCRIPTION OF THE DRAWINGS

FIG. 1 is an external configuration view of a gas measurement apparatus seen from the front side.
FIG. 2 is a perspective view showing a slide structure of the gas measurement apparatus.
FIG. 3 is a perspective view showing the slide structure from the inside of the gas measurement apparatus.
FIG. 4 is an external configuration view of the gas measurement apparatus seen from the rear side.
FIG. 5 is a perspective view showing an inner configuration of the gas measurement apparatus.
FIG. 6 is a partial cross sectional view showing a state of a connection part of a semiconductor gas sensor, a pipe connected to a delivery port, and a second gas sensor.
FIG. 7 is an external configuration view of an attachment attached to the delivery port.
FIG. 8 is a perspective view of the gas measurement apparatus seen from the lateral side.
FIG. 9 is a configuration view of a control system of the gas measurement apparatus.

BRIEF DESCRIPTION OF THE REFERENCE NUMERALS

| | |
|---|---|
| 1 | GAS MEASUREMENT APPARATUS |
| 2 | BODY UNIT |
| 3 | COVER UNIT |
| 5 | BASE UNIT |
| 6 | MEMBER |
| 6A | KNOB UNIT |
| 10 | DELIVERY PORT |
| 15 | PIPE |
| 15A | PIPE |
| 20 | DISPLAY UNIT |
| 30 | OPERATION UNIT |
| 40 | SLIDE PLATE UNIT |
| 40A | STOPPER UNIT |
| 40B | STOPPER UNIT |
| 42 | GUIDE UNIT |
| 44 | SPRING |
| 50 | ABSORBENT HOUSING UNIT |
| 52 | HOLE SECTION |
| 54 | HOLE SECTION |
| 56 | HOLE SECTION |
| 60 | SEMICONDUCTOR GAS SENSOR UNIT (FIRST GAS SENSOR UNIT) |
| 62 | HOUSING |
| 62A | HOLE SECTION |
| 64 | SENSOR CIRCUIT BOARD |
| 66 | SEMICONDUCTOR GAS SENSOR (FIRST GAS SENSOR) |
| 68 | ELECTRONIC COMPONENT |
| 70 | SECOND GAS SENSOR UNIT |
| 72 | SECOND GAS SENSOR |
| 74 | SOLENOID VALVE |
| 76 | PRESSURE SENSOR |
| 80 | MAIN CIRCUIT BOARD |
| 82 | TIMER |
| 84 | ELECTRIC POWER SUPPLY |
| 90 | CPU |

92      ROM
94      RAM
96      NON-VOLATILE MEMORY
98      I/O
100     TUBE

DESCRIPTION OF THE EMBODIMENTS

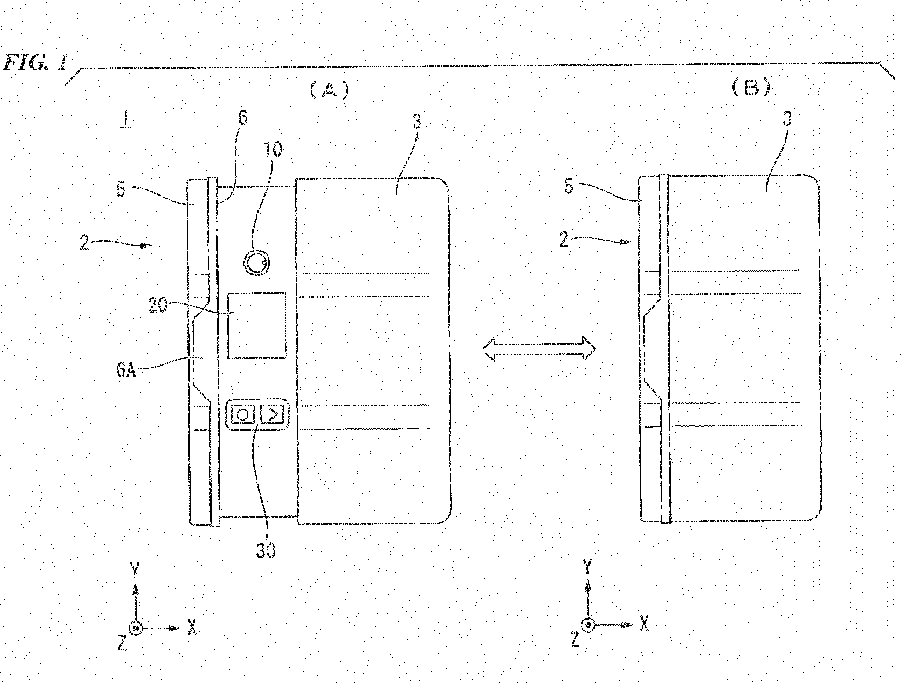

Hereinafter, a gas measurement apparatus (an apparatus for measuring a specific gas component in a user's breath) according to an embodiment of the present invention is described with reference to the drawings. If necessary, an XYZ coordinate system is used for diagramatic representation and explanation. The (A) states of FIGS. 1 to 4 indicate an open state of a gas measurement apparatus 1. The (B) states of FIGS. 1 to 4 indicate a closed state of the gas measurement apparatus 1.

FIG. 1 is an external configuration view of the gas measurement apparatus 1 seen from the front side. The gas measurement apparatus 1 includes a holding structure that holds a cover unit 3 to be any one of an open state and a closed state by an operation to slide the cover unit 3 in the X direction in FIG. 1 by an operator.

The gas measurement apparatus 1 includes a body unit 2 and the cover unit 3 that covers part of the body unit 2 in the closed state. A portion in a first surface of the body unit 2, which is exposed only in the open state, is provided with a delivery port 10, a display unit 20, and an operation unit 30.

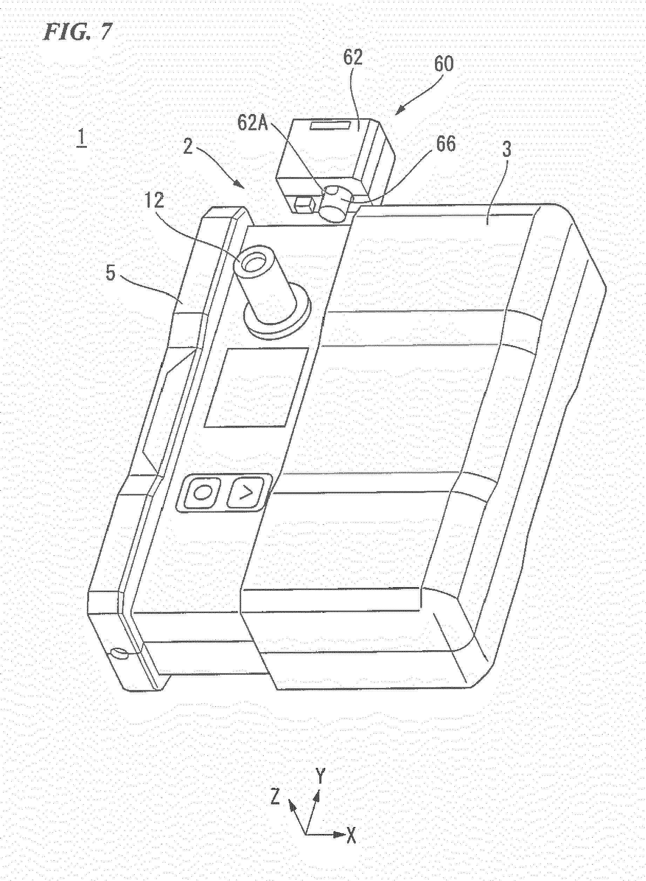

An attachable and detachable attachment 12 (shown in FIG. 7) having a hollow structure is attached to the delivery port 10 in the open state in a state where the attachment 12 is inserted in the delivery port 10. Further, a straw (not shown) is provided on the attachment 12. The breath of the user is delivered to the delivery port 10 through the straw and the attachment 12.

The attachment 12 may have a configuration in which the straw is not provided, and the breath of the user may be delivered to the delivery port 10 through the attachment 12. Further, the delivery port 10 may have a configuration in which the attachment 12 is not attached to the delivery port 10, and the user's breath may be delivered directly to the delivery port 10 without the attachment 12.

The display unit 20 is, for example, a display device such as a liquid crystal display device or an organic electroluminescence (EL) display device. The content of display of the display unit 20 is determined by a CPU 90 described below. The display unit 20 displays a variety of information for the user by a setting screen, a screen showing a measurement result, and the like. The operation unit 30 accepts a variety of operations (on/off of the power, input of user information or the like, scroll of the screen, and the like) by the user. The content of operation applied on the operation unit 30 is output to the CPU 90 described below.

Further, a base unit 5 is formed on the body unit 2 of the gas measurement apparatus 1, the base unit 5 being formed to have a surface form that is consecutive to the cover unit 3 in the closed state. A member 6 that has a form corresponding to an end portion of the cover unit 3 and that comes into contact with the cover unit 3 in the closed state for improving airtightness is attached to an end portion of the base unit 5 that comes into contact with the cover unit 3. The member 6 is formed of an elastic material such as a rubber (for example, a silicon rubber) and an elastomer. A knob unit 6A is provided on the member 6 at a position close to the central portion of the member 6 with respect to the Y direction in FIG. 1 such that the user can easily perform an open operation. The knob unit 6A has a tilt such that a portion of the knob unit 6A is positioned further in the +Z direction as the portion of the knob unit 6A is positioned further in the -X direction in FIG. 1.

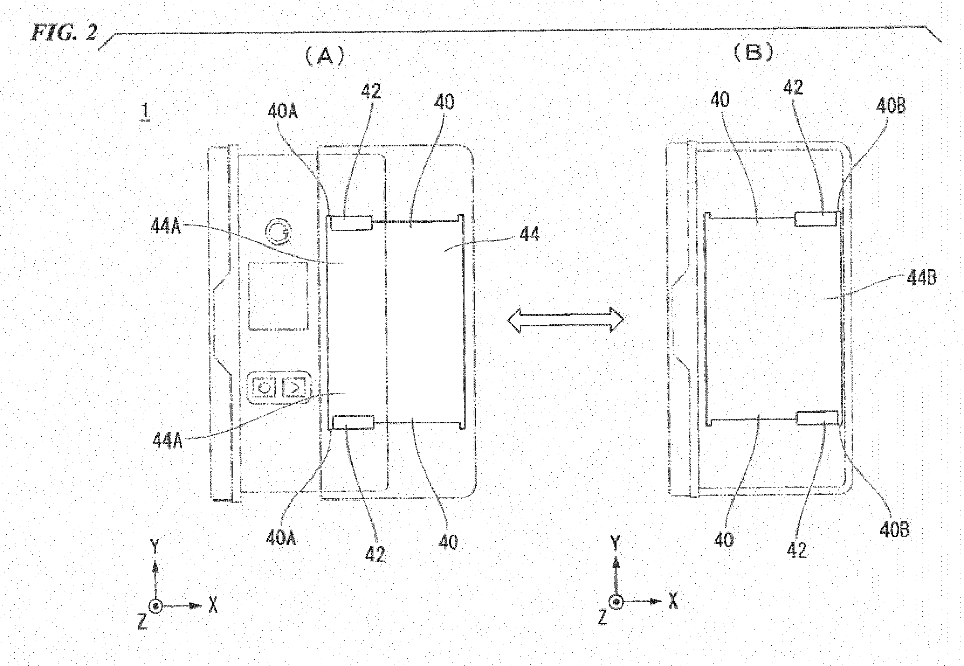

FIG. 2 is a perspective view showing a slide structure of the gas measurement apparatus 1. A slide plate unit 40 is attached to the inside (side invisible from the outside) of the cover unit 3.

The slide plate unit 40 is supported slidably with respect to a guide unit 42, by the guide unit 42 attached to the body unit 2. Stopper units 40A, 40B are formed on both ends of the slide plate unit 40. The stopper units 40A, 40B limit the slide width of the body unit 2 and the cover unit 3. According to such a slide structure, the user can make the gas measurement apparatus 1 be in a desired state of the open state and the closed state by a simple operation. Accordingly, the gas measurement apparatus 1 can improve operability for the user.

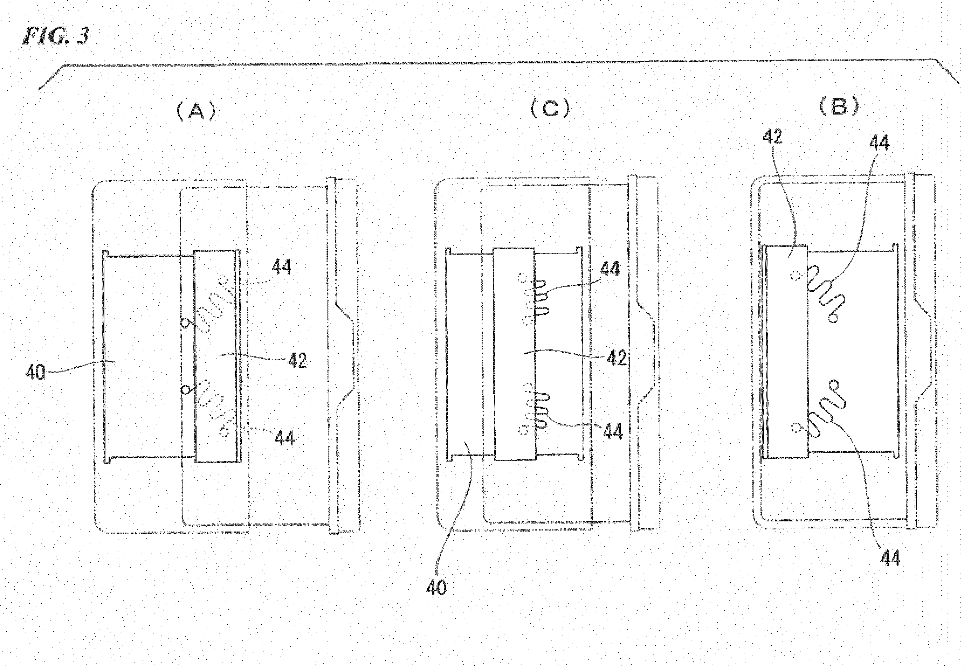

FIG. 3 is a perspective view showing the slide structure from the inside of the gas measurement apparatus 1.

A spring 44 (biasing unit) is provided between the slide plate unit 40 attached to the inside of the cover unit 3 and the guide unit 42 attached to the outside of the body unit 2. One end portion of the spring 44 is attached to the slide plate unit 40, and the other end portion of the spring 44 is attached to the guide unit 42, such that the spring 44 is rotatable centered on each of the attached portions.

In the open state of the gas measurement apparatus 1, the spring 44 exerts a force pushing (biasing) the stopper unit 40A formed on the slide plate unit 40 to the guide unit 42. In the closed state of the gas measurement apparatus 1, the spring 44 exerts a force pushing (biasing) the stopper unit 40B formed on the slide plate unit 40 to the guide unit 42. The cover unit 3 biased to the open state or the closed state stops at a predetermined relative position with respect to the body unit 2 by the stopper units 40A, 40B. Further, the spring 44 forms a configuration such that the direction of the pushing force changes smoothly in an intermediate state of the open state and the closed state when the gas measurement apparatus 1 is slid from the open state to the closed state or from the closed state to the open state. Accordingly, the user can operate the gas measurement apparatus 1 smoothly to the open state and the closed state without the cover unit 3 being rattled. A mechanical lock structure or the like may be provided in place of the spring 44.

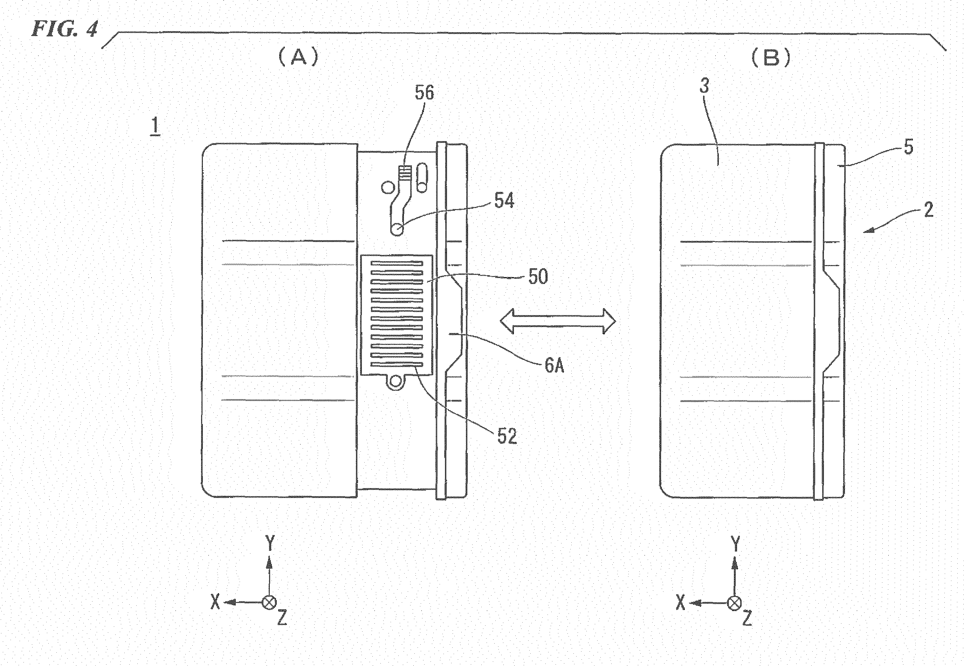

FIG. 4 is an external configuration view of the gas measurement apparatus 1 seen from the rear side. A portion in a second surface of the body unit 2, which is exposed outside only in the open state, is provided with an absorbent housing unit 50, a hole section 52, a hole section 54, and a hole section 56. In FIG. 4, the knob unit 6A has a tilt such that a portion of the knob unit 6A is positioned further in the -Z direction as the portion of the knob unit 6A is positioned further in the -X direction in FIG. 4.

The absorbent housing unit 50 houses a gas absorbent such as activated carbon within the absorbent housing unit 50. In the closed state of the gas measurement apparatus 1, a space that houses activated carbon is connected to a space in which a semiconductor gas sensor 66 (first gas sensor) described below is provided through the hole section 52, a gap between the body unit 2 and the cover unit 3, and the hole section 54 or the hole section 56, to form a closed space. Thereby, during the gas measurement apparatus 1 is in the closed state, a variety of gas components are removed from the closed space in which the semiconductor gas sensor 66 is provided, and it is possible to prevent contamination or degradation of the semiconductor gas sensor 66. The absorbent housing unit 50 may house a gas absorbent such as zeolite, molecular sieve, and silica gel, in place of (or in addition to) activated carbon.

Further, the absorbent housing unit 50 can be connected to the space that houses activated carbon and a space in which a second gas sensor 72 described below is provided to form a closed space, and it is possible to prevent contamination or degradation for the second gas sensor 72.

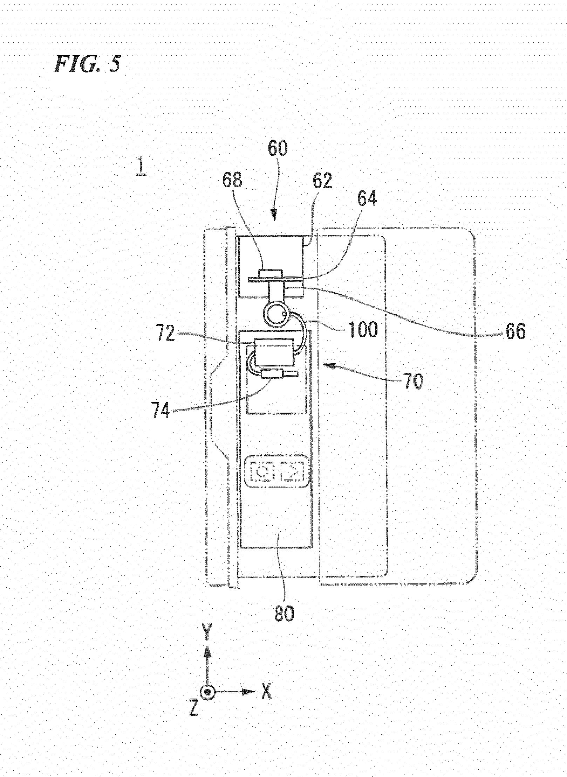

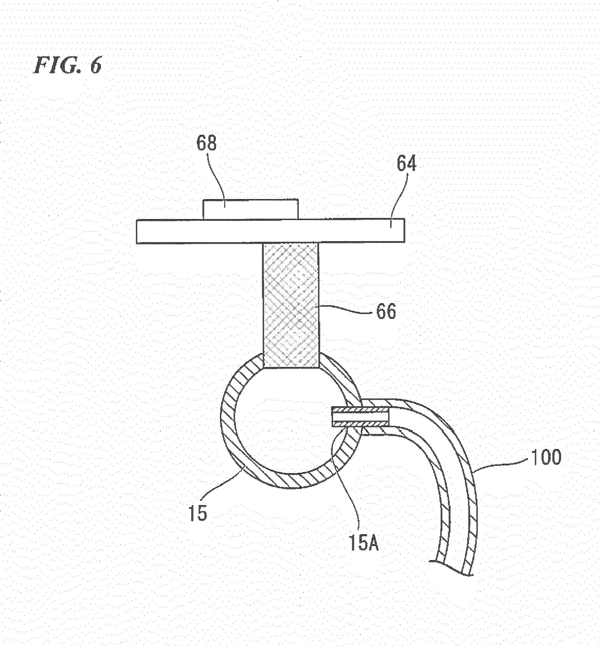

FIG. 5 is a perspective view showing an inner configuration of the gas measurement apparatus 1. A semiconductor gas sensor unit 60 (first gas sensor unit) attachable to and detachable from the body unit 2 (replaceable) is attached to the gas measurement apparatus 1. FIG. 6 is a partial cross sectional view showing a state of a connection part of the semiconductor gas sensor 66, the pipe 15 connected to the delivery port 10, and the second gas sensor 72. FIG. 7 is an external configuration view of the gas measurement apparatus 1 in a state where the semiconductor gas sensor unit 60 is removed. The semiconductor gas sensor unit 60 houses a sensor circuit board 64, a semiconductor gas sensor 66, and an electronic component 68 (for example, storage component) within a housing 62 capable of fitting to the body unit 2. The semiconductor gas sensor 66 is provided on one side of the sensor circuit board 64. The electronic component 68 is provided on the other side of the sensor circuit board 64. The semiconductor gas sensor 66 is provided such that a detection surface protrudes outward of the housing 62 through the hole section 62A provided on the housing 62. A tip portion of the semiconductor gas sensor 66 is inserted in the pipe 15. Tin oxide ($SnO_2$) or

the like is formed on the detection surface of the semiconductor gas sensor 66, and when a detection target gas such as acetone or other interference gas comes into contact with the detection surface, the electric resistance decreases. The semiconductor gas sensor 66 is provided with a heater and an electrode. The semiconductor gas sensor 66 detects the concentration of the detection target gas based on the decrease of the electrical resistance in the detection surface.

The breath of the user includes various types of gases such as ketone body, ethanol, acetaldehyde, and the like. The semiconductor gas sensor 66 shows, for example, a high sensitivity with respect to acetone as a type of ketone body. Acetone is a byproduct of lipid metabolism, and the concentration of acetone in a breath is an index value representing the amount of lipid metabolism. When carbohydrate energy is sufficiently present in the body, fat is not burned, and therefore the concentration of acetone in a breath is low. When carbohydrate energy is insufficient in the body, fat is burned, and therefore the concentration of acetone in a breath is high.

The surface of the body unit 2, the cover unit 3, and the housing of the semiconductor gas sensor unit 60 are formed, for example, by an acrylonitrile butadiene styrene (ABS) resin, polycarbonate, and the like. The member 6 is, for example, an elastic member such as a rubber (for example, a silicon rubber) and an elastomer. The surface of the display unit 20 is formed, for example, by an acrylonitrile styrene (AS) resin or an acrylic resin. The button unit of the operation unit 30 is formed by an ABS resin, silicon, and the like.

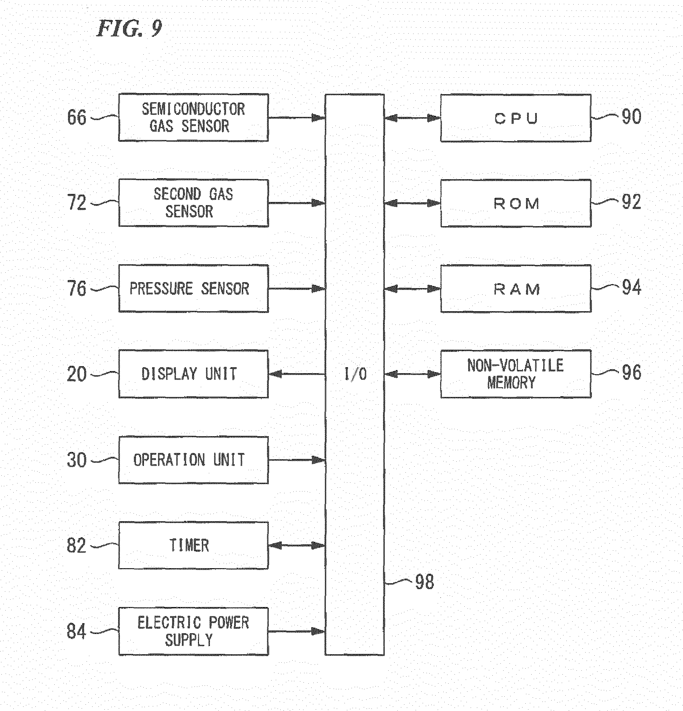

The gas measurement apparatus 1 includes a second gas sensor unit 70. The second gas sensor unit 70 includes the second gas sensor 72 and a solenoid valve 74. A pipe 15A that connects the inner space of the pipe 15 and the outer space of the pipe 15 is provided on the pipe 15. The pipe 15A and the second gas sensor 72 are connected to each other by a tube 100. The second gas sensor 72 is a sensor having a contamination lifetime different from that of the semiconductor gas sensor 66. The solenoid valve 74 operates, for example, after a predetermined time (for example, about several seconds) has elapsed since a pressure sensor 76 (refer to FIG. 9) that measures the pressure of the space inside the gas measurement apparatus 1 connected to the delivery port 10 detects a pressure equal to or greater than a reference value and generates a negative pressure inside the second gas sensor 72. Thereby, the user's breath which is delivered to the delivery port 10 is introduced into the inside of the second gas sensor 72 through the pipe 15, the pipe 15A, and a tube 100. The solenoid valve 74 operates after the predetermined time has elapsed since the pressure sensor 76 detects the pressure equal to or greater than the reference value, and thereby the end breath can be introduced into the second gas sensor 72.

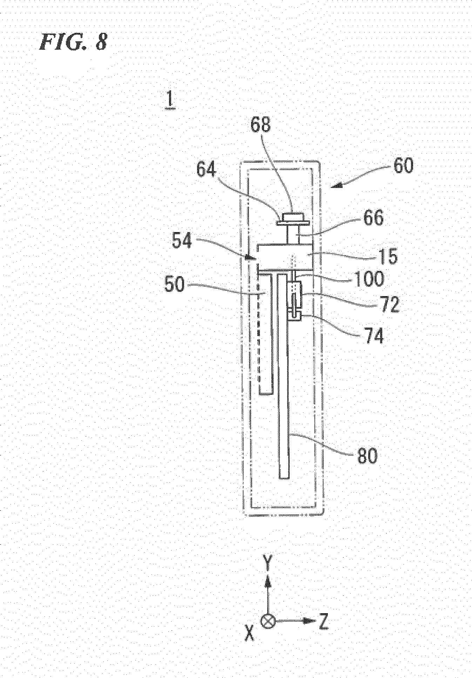

The gas measurement apparatus 1 includes a main circuit board 80. FIG. 8 is a perspective view of the gas measurement apparatus 1 seen from the lateral side (from the -X direction in FIG.5). The CPU 90 described below or the like is provided on the main circuit board 80. FIG. 9 is a configuration view of a control system of the gas measurement apparatus 1. The gas measurement system 1 includes a timer 82, an electric power supply 84, a central processing unit (CPU) 90, a read only memory (ROM) 92, a random access memory (RAM) 94, a non-volatile memory 96, and the like, in addition to the above-described configurations. The configuration elements are communicatably connected through an I/O 98 to one another. The CPU 90 controls each unit of the gas measurement apparatus 1. The CPU 90 performs a process of determining the measurement result of the gas measurement apparatus 1, for example, in consideration of both a detection value of the semiconductor gas sensor 66 and a detection value of the second gas sensor 72. The ROM 92 stores a program executed by the CPU 90 and the like. The RAM 94 functions as a working memory when the CPU 90 performs a process. The non-volatile memory 96 stores the program executed by the CPU 90, data of the user maintained by the gas measurement apparatus 1, and the like.

The CPU 90 derives a measurement result Cone of the gas measurement apparatus 1, for example, based on Expression (1). In Expression (1), C1 represents a detection value of the semiconductor gas sensor 66, and C2 represents a detection value of the second gas sensor 72. k1 and k2 are coefficients, and an optimum value obtained in advance by experiments or the like are used for k1 and k2. k2 is set to, for example, a negative value. In place of Expression (1), the measurement result Conc of the gas measurement apparatus 1 may be derived by a polynomial expression of two orders or more using C1 and C2, and the measurement result Conc of the gas measurement apparatus 1 may be derived by a map using C1 and C2 as coordinates. A parameter (for example, coefficients k1, k2) for deriving the measurement result Conc of the gas measurement apparatus 1 may be adjusted depending on the gender, age, and build of the user.

$$Conc = k1 \times C1 + k2 \times C2 \cdots (1)$$

The gas measurement apparatus 1 of the present embodiment described above includes: the body unit 2 including the delivery port 10 in which the breath of the user is delivered directly or through the attachment 12, the semiconductor gas sensor 66 connected to the inside of the delivery port 10, and the absorbent housing unit 50 that houses the absorbent; the cover unit 3 capable of being operated to be slid in a predetermined direction with respect to the body unit 2 and thereby being set to one of the open state in which the delivery port 10 and the absorbent housing unit 50 are exposed and the closed state in which the delivery port 10 and the absorbent housing unit 50 are cut off from the outside air, and at least in the closed state, the space in which the semiconductor gas sensor 66 is provided and the inside of the absorbent housing unit 50 are connected to each other.

According to the gas measurement apparatus 1 of the present embodiment includes the slide structure shown in FIG. 2 as an example, and thereby it is possible to improve operability for the user. Further, according to the gas measurement apparatus 1 of the present embodiment, in the closed state, the inside of the absorbent housing unit 50 that houses an absorbent is connected to the space in which the semiconductor gas sensor 66 is provided to form the closed space in a state where contamination or degradation causes are removed, and therefore it is possible to prevent contamination or degradation of the semiconductor gas sensor 66. That is, it is possible to improve operability for the user, and it is possible to prevent contamination or degradation of the semiconductor sensor. Further, the absorbent housing unit 50 is also connected to the space in which the second gas sensor 72 is provided to form a closed space "in a state where contamination or degradation causes are removed", and therefore it is also possible to prevent contamination or degradation of the second gas sensor.

Further, according to the gas measurement apparatus 1 of the present embodiment, the open state or the closed state is held by the spring 44 with a loose holding force, and therefore it is possible to prevent rattling when being used.

Further, according to the gas measurement apparatus 1 of the present embodiment, the semiconductor gas sensor unit 60 is attachable to and detachable from the body unit 2 (replaceable), and therefore it is possible to keep the semiconductor gas sensor 66 a fresh state.

Further, according to the gas measurement apparatus 1 of the present embodiment, the member 6 formed of an elastic member such as a rubber (for example, a silicon rubber) and an elastomer comes into contact with the cover unit 3 in the closed state to improve airtightness, and therefore it is possible to further effectively prevent contamination of the semiconductor sensor. Further, according to the knob unit 6A formed on the member 6, it is possible to further improve operability for the user.

Although the embodiment of the invention has been described, the invention is not limited to the above-described embodiment, and a variety of modification and substitution can be added without departing from the scope of the invention.

**Claims**

1. A gas measurement apparatus comprising:

   a body unit including a delivery port in which a user's breath is delivered, a gas sensor connected to the inside of the delivery port, and an absorbent housing unit that houses an absorbent;
a cover unit capable of being operated to be slid in a predetermined direction with respect to the body unit and thereby being set to one of an open state in which the delivery port and the absorbent housing unit are exposed and a closed state in which the delivery port and the absorbent housing unit are cut off from an outside air, wherein
at least in the closed state, a space in which the gas sensor is provided and the inside of the absorbent housing unit are connected to each other.

2. The gas measurement apparatus according to claim 1, comprising:

> a holding structure used to hold the cover unit to be any one of the open state and the closed state.

3. The gas measurement apparatus according to claim 2, wherein
the holding structure comprises:

> a biasing unit that biases the cover unit toward any one of the open state and the closed state from an intermediate state of the open state and the closed state; and
> a stopper unit that stops the cover unit biased by the biasing unit at a predetermined relative position with respect to the body unit.

4. The gas measurement apparatus according to any one of claims 1 to 3, wherein
the gas sensor is replaceable in the open state such that a housing that houses the gas sensor is replaceable.

5. The gas measurement apparatus according to any one of claims 1 to 4, wherein
an elastic member that has a form corresponding to an end portion of the cover unit and that comes into contact with the cover unit in the closed state for improving airtightness is attached to an end portion of the body unit that comes into contact with the cover unit.

EP 3 002 585 A1

FIG. 1

(A)

(B)

FIG. 2

（A）

1

40A 42 40

44A 44

44A

40A 42 40

Y
Z X

（B）

40 42 40B

44B

40 42 40B

Y
Z X

EP 3 002 585 A1

*FIG. 3*

EP 3 002 585 A1

FIG. 4

(A)

(B)

EP 3 002 585 A1

*FIG. 5*

FIG. 6

FIG. 7

*FIG. 8*

*FIG. 9*

| | | |
|---|---|---|
| 66 — SEMICONDUCTOR GAS SENSOR | → | ← I/O → CPU — 90 |
| 72 — SECOND GAS SENSOR | → | ← → ROM — 92 |
| 76 — PRESSURE SENSOR | → | ← → RAM — 94 |
| 20 — DISPLAY UNIT | ← | ← → NON-VOLATILE MEMORY — 96 |
| 30 — OPERATION UNIT | → | |
| 82 — TIMER | ↔ | |
| 84 — ELECTRIC POWER SUPPLY | → | |

I/O — 98

Europäisches
Patentamt

European
Patent Office

Office européen
des brevets

# EUROPEAN SEARCH REPORT

**Application Number**

EP 15 18 7012

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/083527 A1 (FLAHERTY BRYAN [US] ET AL) 21 April 2005 (2005-04-21) * paragraphs [0029], [0030], [0035]; figures 1, 2, 8, 9 * | 1-5 | INV. G01N33/00 A61B5/08 G01N33/497 |
| A | US 5 026 027 A (HAMILTON LYLE H [US]) 25 June 1991 (1991-06-25) * column 3, lines 40-51; figure 5 * | 1-5 | |
| A | US 3 321 976 A (JONES WILLIAM C) 30 May 1967 (1967-05-30) * column 3, lines 5-25; claim 2; figures 1, 2 * | 1-5 | |
| A | US 5 284 054 A (LOEBACH ERNST [DE]) 8 February 1994 (1994-02-08) * column 4, line 58 - column 5, line 17; figures 1, 2 * | 1-5 | |
| A | WO 2008/063404 A2 (BAYER HEALTHCARE LLC [US]; CREAVEN JOHN P [US]) 29 May 2008 (2008-05-29) * paragraphs [0025] - [0027]; figures 2, 3 * | 1-5 | TECHNICAL FIELDS SEARCHED (IPC) G01N A61B |
| A | JP 2010 025717 A (SHARP KK) 4 February 2010 (2010-02-04) * paragraphs [0051] - [0055]; figures 1-5 * | 1-5 | |
| A | JP 2010 107310 A (SHARP KK) 13 May 2010 (2010-05-13) * paragraphs [0058] - [0060] * | 1-5 | |

The present search report has been drawn up for all claims

| Place of search | Date of completion of the search | Examiner |
|---|---|---|
| The Hague | 15 February 2016 | Marzocchi, Olaf |

EPO FORM 1503 03.82 (P04C01)

## ANNEX TO THE EUROPEAN SEARCH REPORT
## ON EUROPEAN PATENT APPLICATION NO.

EP 15 18 7012

This annex lists the patent family members relating to the patent documents cited in the above-mentioned European search report.
The members are as contained in the European Patent Office EDP file on
The European Patent Office is in no way liable for these particulars which are merely given for the purpose of information.

15-02-2016

| Patent document cited in search report | | Publication date | Patent family member(s) | | Publication date |
|---|---|---|---|---|---|
| US 2005083527 | A1 | 21-04-2005 | US 2005083527 A1 | | 21-04-2005 |
| | | | US 2007261472 A1 | | 15-11-2007 |
| | | | US 2009260418 A1 | | 22-10-2009 |
| US 5026027 | A | 25-06-1991 | NONE | | |
| US 3321976 | A | 30-05-1967 | NONE | | |
| US 5284054 | A | 08-02-1994 | AT 113717 T | | 15-11-1994 |
| | | | CH 679886 A5 | | 30-04-1992 |
| | | | DE 59007656 D1 | | 08-12-1994 |
| | | | EP 0491000 A1 | | 24-06-1992 |
| | | | JP H05500114 A | | 14-01-1993 |
| | | | US 5284054 A | | 08-02-1994 |
| | | | WO 9103719 A1 | | 21-03-1991 |
| WO 2008063404 | A2 | 29-05-2008 | CN 101563606 A | | 21-10-2009 |
| | | | EP 2092328 A2 | | 26-08-2009 |
| | | | JP 2010509589 A | | 25-03-2010 |
| | | | US 2010047918 A1 | | 25-02-2010 |
| | | | WO 2008063404 A2 | | 29-05-2008 |
| JP 2010025717 | A | 04-02-2010 | JP 5091039 B2 | | 05-12-2012 |
| | | | JP 2010025717 A | | 04-02-2010 |
| JP 2010107310 | A | 13-05-2010 | JP 5062695 B2 | | 31-10-2012 |
| | | | JP 2010107310 A | | 13-05-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014163671 A